# EUROPEAN PATENT SPECIFICATION

(11) **EP 4 447 872 B1**
(45) Date of publication and mention of the grant of the patent: **14.10.2026**
(21) Application number: 22843872.7
(22) Date of filing: 14.12.2022
(51) Int. Cl.: A61F 2/50, A61F 2/64, A61F 2/68, A61F 2/70

(54) **CURRENT CONTROLLER FOR A MAGNETORHEOLOGICAL ACTUATOR**
STROMREGLER FÜR EINEN MAGNETORHEOLOGISCHEN AKTOR
RÉGULATEUR DE COURANT POUR ACTIONNEUR MAGNÉTORHÉOLOGIQUE

(30) Priority: 16.12.2021 US 202163265544 P
(43) Date of publication of application: 23.10.2024
(73) Proprietor: Össur Iceland EHF, 110 Reykjavik (IS)
(72) Inventor: KRISTMUNDSSON, Jón Trausti, 110 Reykjavik (IS)
(74) Representative: KIPA AB
(86) International application number: PCT/IB2022/062256
(87) International publication number: WO 2023/111920

(56) References cited:
- EP-A1- 1 880 694
- CN-A- 111 685 918
- DE-A1- 19 859 931
- US-A1- 2005 283 257
- US-A1- 2006 074 493
- US-A1- 2019 365 545

## Description

### Field

The present disclosure relates to a magnetorheological actuator used, for example, in a prosthetic or orthotic device, and more particularly to a control system for regulating current circulating in a magnetic coil part of a magnetorheological ("MR") actuator.

### Description

The majority of advanced lower-limb prosthetic devices deployed on lower-limb amputees use a microprocessor-controlled braking device. The braking device's technology and implementation vary significantly, but all provide a certain level of resistance to motion under load, which is leveraged to support the lower-limb amputee while standing on the prosthetic limb. Additionally, where the lower-limb prosthetic device is a knee, the braking device is typically controlled to allow the knee to move freely when the prosthetic limb is in swing phase, hence replicating typical lower limb kinematics observed during gait activities.

Common hydraulic technologies in lower-limb prosthetic devices use valves to provide damping or braking of the device by controlling and regulating the flow of hydraulic fluid. Such hydraulic technologies have a high capacity for quick transition between braking and non-braking states due to the limited displacement required by these components to significantly affect the flow properties, and in turn the hydraulic actuator behaviour. However, one disadvantage of these hydraulic technologies is that they present a rather high restriction to motion, even with the valves fully open, due to the need to move the hydraulic fluid around when the actuator is moving. While these systems are well suited to generating braking forces by creating restriction for the fluid to move through, they present significant inertia and damping when it is required to rapidly move the actuator around under low resistance levels.

In patent publication number US 2005/283257 A1, a prosthetic or orthotic system is disclosed, which includes a magnetorheological (MR) damper.

In patent publication number DE 198 59 931 A1, a leg prosthesisis disclosed with a knee-joint having a damping element for controlling the movement of the knee joint. Further, a control unit for controlling of the damping element is disclosed in dependence on values for an angle of the knee and a force exerted on the prosthesis. A regulating device is also disclosed, which regulates the control of the damping element in dependence on the values that are detected for the angle of the knee and the force exerted on the prosthesis, in accordance with walking characteristics.

In patent publication number US 2019/365545 A1, a variable gain impedance controller is disclosed for use in a control system for controlling a motorized prosthetic or orthotic apparatus provided with a joint.

In patent publication number CN 111 685 918 A, a foot artificial limb device is disclosed with adjustable rigidity.

In patent publication number US 2006/074493 A1, systems and methods are disclosed for loading fluid in a prosthetic knee.

In patent publication number EP 1 880 694 A1, an automated speed-adaptive and patient-adaptive control scheme and system are disclosed for a knee prosthesis.

MR actuators have also been used in lower-limb prosthetics (e.g., prosthetic knees). Use of MR actuators requires accurate control of the braking torque applied at any point in time because failure to generate the appropriate level of braking torque, and to do so in a timely manner, can be highly detrimental to system performance. On the other hand, when properly implemented, use of such actuator technology in a micro-processor controlled prosthetic device has been shown to provide significant benefits at functional, safety and consistency levels.

### SUMMARY

The present invention is defined by the appended claims only, in particular by the scope of appended independent claims. References to "embodiments" throughout the description which are not under the scope of the appended claims merely represents possible exemplary executions and are therefore not part of the present invention.

Shear-type MR actuators present different dynamic characteristics requiring more refined control approaches to optimize their performance range. One particular consideration comes from the time response of the MR actuator. As the braking is achieved through a change in the apparent viscosity of the MR fluid which results in increased friction between the thin discs mounted on the device rotor and stator, achieving a fast response time requires being particularly proactive in driving the current and the resulting magnetic field.

Another phenomenon at play here is the size of the magnetic circuit circulating the flux from the magnetic coil through the MR fluid chamber. Combined with the volume of MR fluid where the change of apparent viscosity needs to take place, this contributes to creating the latency typically observed between the point where the current change is affected and the point where the actuator properties are observed to change in a given set of circumstances.

Another consideration supporting the need for a more proactive current control strategy than what is normally used for controlling current in magnetic coil is the fact that the magnetic coil in a shear-type MR actuator does not behave like a pure theoretical inductor would do. Strongly coupled inductors as found in DC motors or solenoids are known to present well-defined current time-response. On the other hand, magnetic coils integrated in the MR actuators present different characteristics. Failure to properly account for these different characteristics results in the reduction of the actuator performance spectrum, either found as low responsiveness or poor torque generation capabilities.

In accordance with one aspect of the disclosure, a control system for regulating current circulation in a MR actuator used in a micro-processor controlled prosthetic device is provided.

MR actuators are controllable brakes or dampers, in which a MR fluid is subjected to a varying magnetic field, causing a change in the fluid properties, namely the apparent viscosity, which in turn affects the resistive or braking torque generated by the actuator. Varying the magnetic field is typically achieved by varying the amount of current circulating in a magnetic coil, functionally coupled with the actuator, such that the magnetic field variations directly influence the MR fluid state.

In accordance with one aspect of the disclosure, a MR actuator with a current control system is provided. The current control system employs gain scheduling to accelerate changes in current amplitudes. For applications where severe constraints on the MR actuator response time are present, this type of approach advantageously minimizes the time required for the current circulating in the magnetic coil to change, which in turn minimizes the time required for the magnetic field to change, affecting the MR fluid properties and, finally, the actuator braking torque. Such a MR actuator can advantageously be implemented in lower-limb prosthetic devices, like knee joints, which are known to present hard requirements for fast changes in the joint properties, allowing the actuator to properly meet the prosthetic device end-user requirements.

### BRIEF DESCRIPTION OF THE DRAWINGS

Figure 1 is a perspective schematic view of a prosthetic knee design using a shear-type MR rotary actuator.
Figure 2 is a schematic cross-sectional view of a shear-type MR rotary actuator where the high-bandwidth control of the braking torque is achieved.
Figure 3 is a block diagram of a control system for a shear-type MR rotary actuator.
Figure 4 is a detailed block diagram of the control system for a shear-type MR prosthetic knee.
Figure 4A is a block diagram of a phase state machine.
Figure 4B is a block diagram of a sub-phase state machine.
Figure 5 illustrates a block diagram of a high-level control system for a shear-type MR brake actuated prosthetic knee.
Figure 6 illustrates a block diagram of a high-level control system for a shear-type MR brake actuated prosthetic knee, where the magnetic coil current controller uses a Proportional-Integral-Derivative (PID) controller.
Figure 7 illustrates a block diagram of a high-level control system for a shear-type MR brake actuated prosthetic knee, where the magnetic coil current controller uses a Proportional-Integral (PI) controller.
Figure 8 illustrates a block diagram of a high-level control system for a shear-type MR brake actuated prosthetic knee, where the magnetic coil current controller uses a variable gains PI controller.
Figure 9 illustrates a schedule of current controller gains based on the amplitude of the current error for a shear-type MR brake actuated prosthetic knee.
Figure 10 shows graphs comparing the current response to a step input in a MR actuator coil with and without the gain scheduling approach herein described.

### DETAILED DESCRIPTION

Lower-limb support devices (e.g., prosthetic devices, orthotic devices), such as prosthetic knees, have to support a wide variety of tasks in variable environments and a variety of use cases. An actuator of such lower-limb support devices associated with a particular joint advantageously can provide both static and dynamic levels of performance associated with the load and timing requirements of a given task to support the complete range of performance required by human locomotion.

The MR actuator devices, systems and methods herein disclosed make advantageous use of dynamically adjusting the current controller gains in real-time to minimize the time required to reach a set-point value, without having to deal with the negative aspects of running high gains at all times, including when the amplitude (e.g. magnitude) of the error found as the difference between the current set-point and the measured current circulating the magnetic coil is small. Running high gains at all times can cause oscillation of the current circulating in the coil, which can affect the actuator performance and increase energy expenditure (e.g., draining of the battery for the prosthetic device incorporating the MR actuator).

Advantageously, a closed loop current regulation is used at all times in the MR actuator and does not rely on heuristic decision making to decide whether to apply the control scheme or not. Current regulation under closed loop control was previously only used when the error between the current set-point and the measured current was found to be larger than a predetermined threshold. When the error was smaller than the predetermined threshold, closed loop control was not used, and the current set-point was directly fed through to the coil driver circuitry. Additionally, no particular attention was directed at controlling the current when the current set-point was observed to be reducing.

The embodiments herein described advantageously leverage the benefits of the approach while both increasing or decreasing the current set-point, resulting in a uniform response. Additionally, as mentioned above, the current is regulated under closed-loop control at all times, which also contributes to uniformizing actuator dynamic response in all circumstances. This implementation also advantageously makes possible the use of a recovery current pulse in order to reduce the fall time when the current set-point is reduced from a high current value to a negligible value. In addition to the fundamental benefits of the embodiments disclosed herein, additional benefits arise from the use of a new hardware and firmware platform. Particularly, in one implementation the use of a field programmable gate array ("FPGA") to implement the current controller itself allows higher control bandwidth, independently without affecting the resources required to implement the remaining firmware functions. In that context, it is possible to execute the closed-loop current control scheme at a base frequency of 28kHz, instead of the maximum of 5kHz that was achieved in previous implementations of the MR actuator low-level control scheme. Increasing the controller frequency directly improves the performance of the current regulation, as the controller achieves higher bandwidth, which better matches the dynamic of the magnetic coil.

Finally, the embodiments disclosed herein also allow for the use of adjustable gains, as well as configurable duty cycle parameters. Adjustable gains can be used to customize each actuator in case the dynamic response is observed to vary between units in serial production. Similarly, gains or duty cycles values can be configured to uniformize actuator gain during production activities and ensure high repeatability, for example, of the knee joint braking performance from one device to another. Such high level of inter-device repeatability is important for the case of bilateral amputees or when users are replacing their worn unit with a new one, as the perceived consistency in performance is typically associated as a quality marker by the end users.

In addition to the benefits introduced above, configurable duty cycles can also advantageously be adjusted based on battery voltage, accounting for the progressive reduction of battery voltage with use of the prosthetic device. Hence, increasing the magnetic coil drive circuitry duty cycle while the battery voltage is reducing increases the average voltage applied on the magnetic coil, which in turn makes it possible to maintain the maximum magnetic coil current at a steady level and keep the knee joint actuator braking performance fairly uniform throughout the battery discharge cycle. Again, this advantageously improves the user experience through more consistent knee joint performance, which is perceived as a quality marker. Compensation of the battery voltage drop during discharge is particularly interesting when using a lithium battery to power the prosthetic device. As this particular battery chemistry is known to present a fairly large voltage drop between the fully charged voltage and the nominal discharge voltage, selecting a baseline magnetic coil drive circuitry duty cycle at the battery nominal voltage that ensures that sufficient braking torque is achieved, and scaling it down for voltages comprised between the nominal and fully charged voltage then ensures a consistent braking level.

While actuator braking performance and consistency is optimized using the duty cycle scaling scheme introduced above, it artificially reduces the peak braking torque that can be obtained from the system, as the maximum duty cycle would normally not be used at maximum battery voltage. While adequate selection of the required braking torque at nominal battery voltage should not require the use of additional braking torque, certain circumstances sometimes encountered during locomotion activities may call for a sudden increase of the braking torque over its typical maximal level. For example, in case of stumble, or in order to prevent a fall, or when the user is carrying an excessively heavy object, the duty cycle adjustment scheme introduced above advantageously provides peak braking torque that can exceed the typical maximum braking torque by deviating from the normal adjustment to battery voltage and using the maximum duty cycle with no respect to the actual battery voltage.

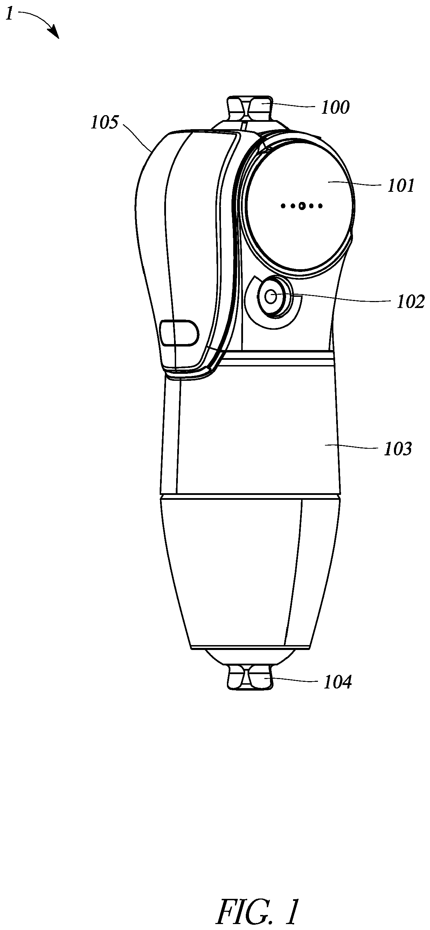

Figure 1 shows an embodiment of a microprocessor-controlled knee prosthetic device **1** where a shear-type rotary MR actuator is used. This embodiment is a modular component targeted at being assembled with other modular components to create a complete prosthetic leg. Proximal Connector **100** is used to structurally connect the knee prosthetic device with the residual limb attachment system (not shown). The knee prosthetic device embodiment is non-specific to the type of residual limb attachment system. Socket systems relying on vacuum or mechanical fastening (e.g., pin liner suspension system), or osteo-integration adapters are non-limiting examples of possible interface solutions. The prosthetic knee integrates the rotary shear-type actuator **101** in the knee rotation axis area, allowing the thigh mounted segment and proximal connector **100** to rotate with respect to the shank segment **103.** Shank segment **103** is used to house the electronic components and battery required to implement the control electronics, actuator driver, on-board sensors and other various support systems that require protection from the environment and possible impact with objects. Mechanical lock actuator **102** enables locking the motion between the thigh and shank segments of the prosthetic knee. Shank segment **103** is structurally connected to the distal connector **104,** which allows connection of the distal modules of the prosthetic leg assembly, namely the shank pylon, as well as the foot and ankle modules. Knee pad **105** is mounted at the front of the MR actuator **101** and the upper part of the shank segment **103** in view of providing protection of the components when the device is used for kneeling or other similar uses.

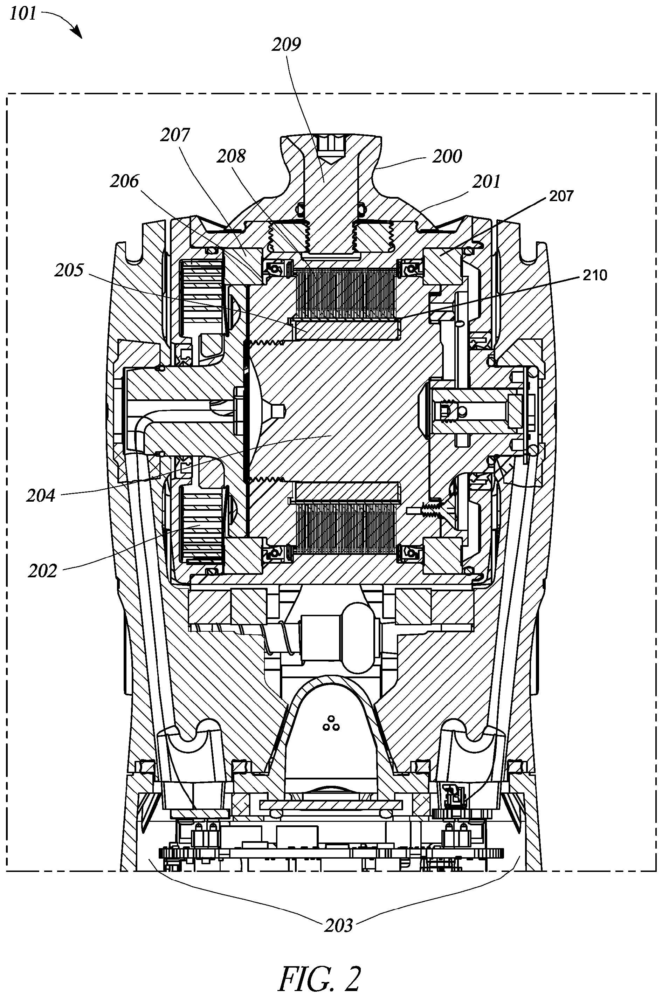

A cross-sectional view of a knee-axis mounted shear-type MR actuator **101** is detailed in Figure 2. Proximal connector **200** is connected to the outer spline **201,** which can rotate with respect to the knee axis and the structural supports **203** using the bearings **207.** Extension spring **202** is operationally connected between the outer spline **201** and the structural supports **203,** allowing to bias the joint towards full extension, meaning that the shank and thigh segment of the prosthetic knee will be forced in a full extended knee configuration by the extension spring **202.** Proximal connector **200** also integrates the actuator filling plug **209,** which can be removed to fill the actuator cavity with MR fluid. The actuator cavity can have a rectangular cross-section revolved around the knee axis, where evenly spaced-out blades manufactured out of magnetic material **208** are alternatively connected to the thigh segment through the outer spline **201** or the shank segment through the inner spline **210** and the core sides **206.** The MR fluid fills in the gap left between the evenly spaced-out blades. The inner spline **210,** assembled to the core sides **206** and the core rod **204** creates a hollowed-out cylindrical region around the knee axis of, for example, rectangular cross-section, which is filled with the magnetic coil **205.**

When current circulates in the magnetic coil **205,** a magnetic field is created and induced in the core rod **204,** the core side **206** and through the actuator cavity. Based on the magnetic field intensity across the actuator cavity, the MR fluid (not shown) changes viscosity and increases resistance to motion between the blade sets **208.** Conversely, when the current is removed, the magnetic field disappears and the fluid viscosity returns to its original state, allowing the blade sets **208** to move relatively to each other with limited resistance to motion. In certain embodiments, it is also possible to use a recovery current pulse, which allows removing the magnetic field faster, by actively driving the current in the opposite direction for a finite amount of time. The direction in which the current circulates in the magnetic coil is irrelevant, as either direction will cause the desired change of apparent viscosity in the MR fluid, but for improved performance over time and usage, regular changes in the current direction are performed, preventing the onset of remanence and its subsequent build up. Onset and build up of remanence in the actuator are observed to negatively affect the actuator performance as it reduces the range of apparent viscosity possible to achieve with the MR fluid, preventing use of the low apparent viscosity properties of the MR fluid and leading to a permanent braking torque being generated by the actuator, even when no field is generated by the magnetic coil. In the case of a prosthetic knee joint, loss of the low apparent viscosity range provided by the MR fluid impacts the no-load speed of the joint, which is critical for proper operation in swing phase of locomotion gait.

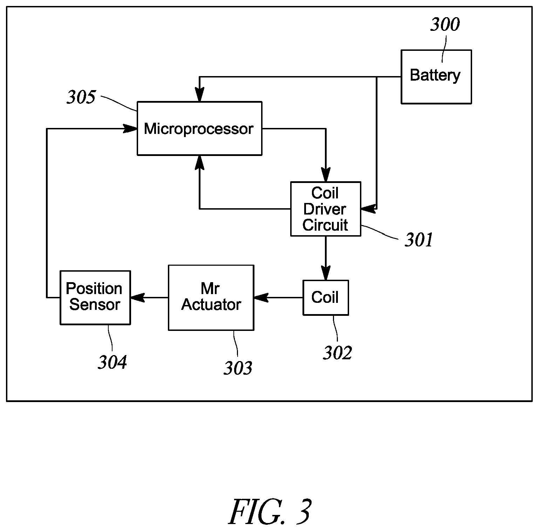

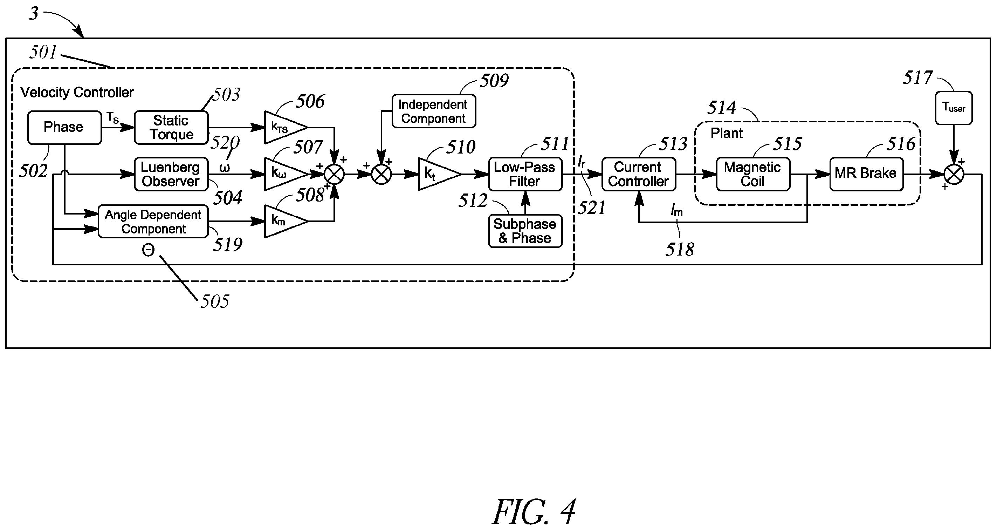

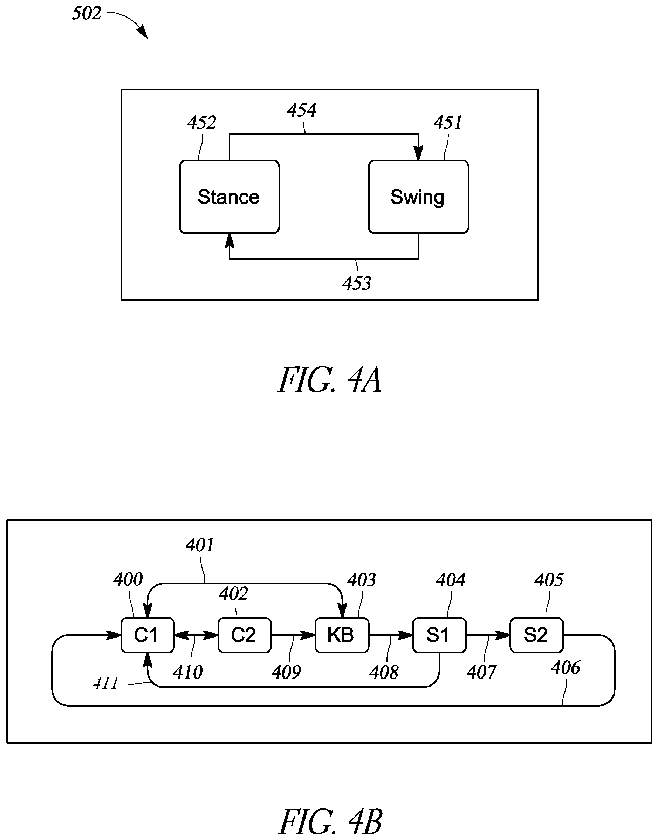

Figure 3 presents a block diagram of the typical arrangement of components for controlling braking torque in an advanced prosthetic knee using a MR actuator. To power the microprocessor **305** and other electronics components, a battery **300** is integrated in the device and is leveraged in the control system implementation. Battery **300** provides power to the microprocessor and associated sensors and housekeeping functions, as well as to the magnetic coil **302** through the coil driver circuit **301** in order to create a magnetic field in the MR actuator **303** and change the braking torque amplitude in the actuator. Microprocessor **305** is in operational communication with the coil driver circuit **301,** commanding the current level and direction of current circulating through the coil to be applied to the coil, while at the same time reading out the actual current level present in the coil. Closed-loop current control is established from the operational connection of the microprocessor **305** and the coil driver circuit **301,** as further detailed in Figure 4. Outer loop actuator control is implemented through the addition of the position sensor **304** which allows measuring the actuator reaction when submitted to external perturbations or loads from the user's residual limb, combined with the braking resistance generated by the MR actuator. Again, the microprocessor **305** is used to interface the position sensor **304** and implement a digital regulation loop, where the actuator velocity is compared to predefined set-points and used to calculate the amount of current to drive the magnetic coil **302** with. While the use of an outer velocity control loop has been shown to provide satisfactory control of a MR actuator in prosthetic knee joints used for typical locomotion activities, this is not an enabling requirement for the deployment of the embodiments herein disclosed. Use of other types of control schemes aligned with the biomechanical requirements of the knee joint in human locomotion is also possible and would result in a similar functional relationship with the current control loop, as any type of outer control loop would be used to define the current set-point to be provided to the closed loop current controller. Amongst a rather large range of options, impedance controller, position controller, torque controller and their combination are viable options for implementation of the outer control loop in the type of applications herein considered.

Figure 4 presents the high-level actuator control scheme block diagram, or outer loop control scheme or system **3.** In one non-limitative embodiment, the outer loop control scheme **3** is implemented as a combination of pure velocity control and some additional heuristic component, which is herein referred to under the general nomenclature of velocity controller **501.** The outer loop control scheme **3** is implemented as a digital controller in the microprocessor **305** and is aimed at managing the MR actuator's behavior and responses to optimally support locomotion tasks the user is attempting. The MR actuator itself is represented by the plant **514,** which is further broken down in magnetic coil **515** and MR brake **516** blocks. While the magnetic coil **515** is normally directly embedded in the MR brake **516,** representing them separately simplifies the control system modeling, even if it is less representative of the physical embodiment. The knee prosthetic as a whole is also subject to interaction with the user and the environment, which are represented as a perturbation torque **517.** The combination of the perturbation torque **517** with the knee prosthetic and actuator-generated braking torque are the target of the control scheme herein presented, as it represents the overall device response when used, which is herein represented as a resulting knee position **505.** The resulting knee joint position is measured by position sensor **304** and is fed back to the outer loop control scheme **3.**

MR brake **516** provides a controlled amount of resistance to motion based on the magnetic field strength going through the MR fluid. Magnetic field is induced by the magnetic coil **515** depending on the amount of current circulating in the magnetic coil. While MR brake **516** resistance is generated in open loop, magnetic coil is controlled in closed loop, through regulation of the current, which is performed through a current controller **513,** such as a digital Proportional-Integral (PI) controller, implemented in either the device embedded microcontroller or any other digital processing platform available on the device. The current controller **513** compares the current measured in the magnetic coil (*Iₘ*)**518** with the current set-point requested from the outer loop controller (*Iᵣ*)**521** to determine how to adjust the command provided to the coil driver circuit **301** to ensure that the measured current *Iₘ* **518** matches the requested current *Iᵣ* **521**. Adjustments to the command signal to coil driver circuit **301** are directly performed by the current controller **513**, where the gains are adjusted to ensure that the correct dynamic response will be obtained from the current controller when the set-point is modified by the outer loop controller. In one non-limitative embodiment, the coil driver circuitry **301** is implemented using a pulse-width modulation scheme, where the duty cycle during which the magnetic coil is connected to the battery supply is proportionally adjusted based on the amplitude of the current command issued by the current controller **513,** leading to an increased average current in the magnetic coil when the current command is increased.

The resulting combination of the MR actuator resistance to motion, user interaction and perturbation torques from the user and the environment result in the prosthetic knee joint instantaneous position Θ **505**. Actuator position data Θ **505** is fed into a Luenberg Observer **504** to generate an actuator rotational velocity estimate ω **520**, which is then fed through the velocity gain k_{ω} **507.** Velocity term of the control loop is the main continuous control part, as a strong correlation exists between actuator velocity and the required actuator resistance level required for the user to successfully complete the locomotion task undertaken. To support the specific actuator behaviours, three discrete terms are added to the control loop and are executed based on specific conditions associated with the device usage (i.e., gait phases and subphases, **502** and **512**). The Static Torque **503** block consists of a feedforward term which is fed through a static torque gain k_{TS} **506** that allows forcing a minimum actuator resistive torque when the actuator velocity is null or below a certain threshold, while the knee prosthetic enters the stance phase following user weight transfer over the prosthetic foot. While the characteristics of the static torque profile can be varied based on the exact nature of the locomotion task, a general characteristic where amplitude is observed to decay in the time following triggering is considered optimum as it provides a better loading response to the user weight without preventing the user from quickly moving the knee if desired. In a situation where the actuator velocity would be sufficiently high, the velocity arm of the controller (Luenberg Observer **504** and velocity gain **507**) would provide enough responsiveness and there is not hard need to add the contribution of the static torque **503** component. Static torque triggering is driven by the Phase detection engine **502,** which is part of the intent management section of the control system of Figures 4A and 4B.

Figure 4A provides details on a Phase state machine **502.** From a human locomotion perspective, two phases are to be considered and are fundamental to the control of the lower-limb prosthetic device. The state machine is built around having a specific state for each phase. Swing Phase state **451** is characterized by the fact that the prosthetic limb is not carrying the user weight or no contact between the prosthetic limb and the ground is observed. For practical consideration, this state will be entered when a pre-defined set of conditions are met, typically coming from the Stance Phase state **452.** Stance Phase state **452** is effectively the counterpart of the Swing Phase state **451** and is characterized by the prosthetic lower limb being in contact with the ground or user body weight being carried by the prosthetic limb. Similar to the Swing Phase state **451,** Stance Phase state **452** will be entered when a predefined set of conditions are met, typically coming from the swing phase state **451.**

On system initialization, the phase state can be defined as either Stance Phase state **452** or Swing Phase state **451.** Once new data is issued by the sensors (e.g., of the knee prosthetic device **1**), state will be re-evaluated and the state machine will directly transition to the correct state by comparing the sensor data to the pre-established transition conditions for stance to swing transition **454** or swing to stance transition **453.** Due to the low latency required for the system to load new sensor data and the Phase state to be evaluated, there is not a hard functional limitation in having the phase considered stance or swing by default. In one non-limiting embodiment of the phase state machine, the swing to stance transition **453** is based on the comparison of the axial load applied on the lower-limb prosthetic device (e.g., of the knee prosthetic device **1**) and measured by the on-board sensors with pre-defined threshold. For example, if the load cell measures an axial load superior to 8 kg, the state machine will make or identify the current state to be stance. Conversely, if the load applied on the lower-limb prosthetic device is measured to be less than 8 kg, the phase will transition to swing. A variety of sensor embodiments can be used to support the phase state machine operation. To name a few, loadcells, accelerometers, pressure sensors, displacement sensors, radar, inductive sensors, and resistive sensors can be leveraged to support the phase state machine. Additionally, stance to swing transition **454** and swing to stance transition **453** can be based on decision mechanisms other than the single thresholding comparison provided as example above. Decision making processes such as multiple thresholding or majority voting could also be applied to define the transition criteria. Additionally, the various methods for deciding if the conditions are met for allowing the system to transition from one state to the other in the phase state machine could also consider multiple data streams at the same time or use sensor integration or fusion to build data stream containing data at a higher level of abstraction.

While the stance and swing phase are characteristic of human locomotion, they do not provide a very detailed segmentation of the behavior required by a lower-limb prosthetic, as this phase segmentation only describes the general configuration of the lower-limb and not the behavior of the various joints in a single phase. To achieve a level of gait control refinement as required by the end user to achieve consistent, stable, and safe locomotion, additional granularity is required, such that the actuator behavior can also be adjusted in a more granular manner.

Figure 4B presents a subphase state machine embodiment that is particularly well suited for controlling knee prosthetic devices, such as the knee prosthetic device 1 shown in Figure 1. While the number and the nature of the subphases can vary, the general approach is typically to consider the subphases required to properly address the requirements of the level walking activity and then to use these subphases, or a subset of them, to address the other locomotion activities, which can typically be addressed satisfactorily with a reduced number of subphases. While a lower number of subphases may make sense for devices using an actuator subsystem not allowing for refined controllability, and a higher number of subphases may increase the complexity of the control system without bringing any benefits to the overall performance, an appropriate number of subphases will allow optimal use of the actuator controllability and address the specific biomechanical requirements of the gait activity in a satisfactory manner. In the specific case of the subphase state machine presented in Figure 4B, use of 5 subphases is aligned with the general understanding of the knee role in level walking as described in modern biomechanical analysis of the lower limb.

C1 subphase **400** represents the Contact 1 subphase of the gait cycle and consists of the level walking stance phase state starting from the occurrence of the contact of the prosthetic limb foot with the ground and where the knee is typically observed to flex under the weight of the end user. This flexion is typically observed to provide a certain level of shock absorption following the occurrence of the contact between the prosthetic foot and the ground surface. To properly support the end user weight transfer to the prosthetic limb, the prosthetic knee actuator (such as actuator **101** in FIGS. 1-2) is required to provide braking torque or torque resisting the motion imposed to the knee joint by the end user weight and momentum.

C2 subphase **402** represents the Contact 2 subphase of the gait cycle and consists in the level walking stance phase state where the knee is observed to extend, following the completion of the C1 subphase flexion motion. Extension motion occurs from a combination of the end-user forward momentum and the end-user residual limb hip extension. Proper support of the knee extension motion by the prosthetic knee actuator (such as actuator **101** in FIGS. 1-2) requires an appropriate amount of resistive torque. Failure to provide enough resistive torque will let the knee quickly extend and hit the extension motion stop, which causes discomfort for the end user. On the other hand, providing too much resistive torque will slow down the extension motion and require the end user to put excessive force to extend the knee.

KB subphase **403** represents the knee break or pre-swing subphase of the level walking gait cycle and consists of the stance phase state where the knee joint is prepared to transition to swing phase. In that part of the level walking stance cycle, the user starts unloading the prosthetic limb and the residual limb thigh segment starts moving into hip flexion, after having reached maximum extension. Proper care must be directed in this subphase actuator control to allow for a smooth transition to swing, without hindering the end user hip flexion, requiring the end-user to lift themself up on their sound foot (i.e., hip-hiking) or causing the prosthetic foot to stick to the floor and breaking the forward momentum of the end-user (late stance locking). To ensure proper synchronism with the user's motion and allow the user's motion to control the whole KB subphase, the prosthetic knee actuator (such as actuator **101** in FIGS. 1-2) is typically placed in a low resistance to motion state, which allows the end-user to properly control the knee joint behavior in this subphase.

S1 subphase **404** represents the Swing 1 subphase of the level walking gait cycle and consists of the swing phase state where the knee joint is actively flexing while not in contact with the ground, allowing to clear the ground while the lower limb is brought back in the proper configuration for the following step. Knee flexion under the momentum imparted to the shank segment through the thigh segment acceleration requires the prosthetic knee actuator (such as actuator **101** in FIGS. 1-2) to present low resistive torque. Failure to properly leverage the residual limb momentum is typically observed to cause a slow flexion movement, which in turn is likely to cause toe-stubbing or fail at generating sufficient ground clearance while the hip is flexing the thigh segment. In both cases, end-user forward progression dynamics will be interrupted, creating a risk of fall or stumble, or reducing the overall efficiency of the walking gait pattern.

S2 subphase **405** represents the Swing 2 subphase of the level walking gait cycle and consists in the swing phase state where the knee joint is actively extending while not in contact with the ground, allowing to fully extend the knee joint in preparation for the upcoming transition to stance phase. Again in this subphase, it is required for the knee joint to achieve sufficient velocity to ensure that the limb is fully extended ahead of the moment where the prosthetic foot would contact the ground surface, while also managing the joint velocity profile in such a way as to avoid the knee from hitting the extension stop, which is uncomfortable for the user and could negatively affect the end-user capacity to transfer weight to the prosthetic limb in a timely manner. Properly supporting the S2 subphase requires the prosthetic knee actuator (such as actuator **101** in FIGS. 1-2) to present a low resistance state for most of the subphase, followed by an increase of the actuator resistance to motion to smoothly decelerate the joint in the last part of the extension motion. This type of actuator behavior can be obtained by using the subphase information combined with the knee position sensor data in the actuator control scheme.

C1 to C2 state transition **410** occurs when the knee joint behavior is observed to transition from stance flexion to stance extension, under the influence of the end-user. This transition is typically triggered through monitoring of the knee joint velocity direction and typically occurs from C1 to C2 during level walking. It is possible however that transition in the opposite direction (i.e., C2 to C1) could be observed in atypical circumstances or in other activities where knee joint extension is followed by knee joint flexion while in stance phase. Interruption in the level walking stance phase gait cycle could cause the extension motion to stop and the knee to start flexing again. Similarly, sudden stopping due to the presence of an obstacle or losing balance over the prosthetic foot could also generate this type of transition. On the other hand, other activities can be managed through these subphases and provide a more visual example of the bidirectionality of this specific transition. For example, stand to sit transfer is typically supported directly through the C1 subphase, since the knee is flexing in stance phase. Conversely, sit to stand transfer is typically directly supported through the C2 subphase. Since these transfer activities can be performed in any sequence, with or without really reaching the standing and sitting end points, this illustrates the need to support this transition in a bi-directional manner.

C2 to KB state transition **409** occurs when the user is observed to be ready to initiate the transition to swing phase and is again triggered through monitoring of the embedded sensors. This corresponds to the mid-stance to late-stance part of the level walking gait cycle, where the user center of mass is observed to have moved anteriorly to the prosthetic foot and the hip is about to start flexing. Identification of this particular configuration of the user segments and body dynamics can be achieved by making sure that the knee is in an extended position and shows low velocity, indicative that the C2 subphase has really completed. Additionally, monitoring that the knee joint shows sufficient extension moment allows to estimate the end user center of mass to be located anteriorly to the prosthetic foot. Finally, monitoring that sufficient shank sagittal plane rotational velocity is observed allows to ensure that the user is actually showing forward progression momentum while rolling over the toe of the prosthetic foot and/or has started to flex the residual limb hip. As this transition is a rather dynamic one and actually requires the knee joint to be fully extended, it is typically considered as a one-way state transition. Exiting the KB state to return to an earlier stance phase state in level walking requires the knee to flex, which then matches the C1 subphase, and not the C2 subphase.

KB state **403** normal exit transition pattern during level walking gait is observed to be the KB to S1 state transition **408.** This transition is observed to take place when the user has completed the weight transfer to the contralateral limb, leaving the prosthetic limb now in swing phase. This transition is managed through the combination of two conditions monitored from the sensor data stream. For one, the loadcell signal has to indicate that the load on the prosthetic limb has dropped below the threshold value used to determine the phase. In other words, the phase has to be detected as being swing. Additionally, to prevent the occurrence of a false positive detection of a KB to S1 transition, a minimum flexion angle is required for the state transition to be executed. In a non-limiting embodiment, an angle of 15° is used. Use of the additional minimum flexion angle condition over simply using the threshold on the loadcell measurement provides the added benefit of helping debounce the loadcell signal and makes sure the user has committed to the transition before actually implementing the state change (i.e., residual limb hip flexion has started to take place, causing the knee to flex). Debouncing is required for cases where the load transferred to the ground through the prosthetic limb would be moving across the threshold value with small amplitude, causing the system to quickly cycle through the stance and swing phases. Use of the minimum angle threshold minimizes that concern as selection of a correct threshold value allows to make sure that the user has started flexing the residual limb, which greatly removes the capacity to be loading the prosthetic limb.

KB to C1 and C1 to KB bidirectional state transition **401** integrate two specific cases that are not typically observed during level walking gait but are often encountered in the general user population based on the high variability of the environment in which human locomotion takes place, causing deviation in the normal occurrence of gait events. Firstly, C1 to KB state transition is in fact equivalent to the C2 to KB state transition introduced above and would typically be observed in cases where the C1 to C2 transition would not be observed, mainly caused by the knee extension not taking place as expected, but where KB transition is still observed to take place. For example, in the case where the user would enter C1 with a fully extended knee, the knee would not generate enough flexion torque to cause the knee to yield, or flex, under load and would not allow it to meet the conditions to enter C2 from C1 but could still meet the conditions for the KB transition in late stance. This type of situation is often observed when users were trained to use simpler mechanical prosthetic knee technology, where they have to force the knee in hyperextension during stance phase to compensate for the device's lack of capacity to provide support.

Secondly, KB to C1 state transition is required to provide a recovery mechanism to the end user and pre-empt some falls from being generated by the user failing at properly progressing through the normal gait events and subphases. Two main situations are observed in level walking where the KB to C1 transition is leveraged and are both associated with a general situation where the observation of the sensor stream indicates that it would be safer for the end-user to have the knee provide support instead of the no-support joint behavior associated with the KB state. In one case, this transition would be triggered by the observation of the knee extension moment being too small and the knee joint flexion angle being too large. This situation represents a case where the end-user center of mass progression has stopped while KB was previously entered, leaving the center of mass posterior to the prosthetic foot and the end-user in an unstable position. This could be observed if the user is required to abruptly stop, or get pushed back, for example. In the other case, if the user loading of the prosthetic device is observed to increase while KB was already entered, this is again indicative that the end-user center of mass is not progressing forward as expected and the user is not transferring their weight away from the prosthetic limb like normally observed in KB.

S1 to S2 state transition **407** occurs when the knee joint has completed the level walking swing phase flexion motion and is observed to start extending. This inversion of the knee rotation direction is normally synchronized with the residual limb hip flexion motion completion and will position the lower limb in a configuration that is ready to support the coming prosthetic foot contact with the ground. This state transition is managed through the monitoring of the knee joint velocity direction, which is implemented through using a null velocity threshold to decide whether the current direction of motion has reached the value allowing for the transition to take place.

S1 to C1 state transition **411** is observed to occur in specific circumstances where sufficient load would be observed on the prosthetic device, through monitoring of the loadcell sensor read out, while is the prosthetic device is expected to be operating in swing phase. Occurrence of stance phase detection during swing flexion can be related to a variety of factors but all indicate a disruption of the normal level walking gait cycle requiring providing support to the end-user and preventing the knee from collapsing under the user. For example, presence of an obstacle interrupting the swing flexion motion and/or interrupting the end-user forward progression momentum could easily result in this transition taking place. Obstacles such as curbs, shrubs, tall grass, or snow are typical examples of obstacles encountered in daily living. Similar to the KB to C1 case previously introduced, situations where the user would be pushed back (e.g., from pushing against a heavy door, or bumping into someone) are also cases where this transition would be observed to occur. Again, reverting the subphase to C1 ensures that the knee will be providing resistance to flexion load, preventing its collapse and subsequent user fall.

S2 to C1 state transition **406** is observed to occur as the level walking swing phase extension knee joint motion completes and the prosthetic foot enters in contact with the ground again, registering load on the prosthetic limb again. Detection of the loadcell sensor signal exceeding the predetermined threshold causes the phase to transition back to swing and the subphase to go back to the C1 subphase, allowing the whole cycle to start again.

With reference to FIG. 4, information provided by phase detection engine **502** is also fed to the Angle Dependant Component block **519,** along with the actuator position **505** information. Similarly, the Angle Dependant Component **519** is a feedforward term which is fed through a gain kₘ **508** applied during stance phase but where the characteristic of the contribution of this arm of the controller is scaled according to the actuator position **505.** Typically, but not to be considered as a limitative embodiment, this feedforward contribution is shaped like a saw tooth waveform, providing an increasing contribution over approximately the first half of the actuator motion range, before decreasing back to its original level over the second half of the actuator motion range. This contribution provides a base level actuator resistance torque which is scaled up depending on actuator angle when the prosthetic knee is operating in stance phase. This is observed to reduce the actuator performance dependency on the flexion-extension actuator velocity, which allows for more consistent support of the user's weight with increasing flexion angles and during motion direction changes, where velocity goes through a null point and where the user could be left without actuator resistance while loading the actuator. Additionally, the Angle Dependant Component **519** feedforward term can be defined in such as way as to compensate for the increased actuator loading from the user as the angle increases, which causes an increase of the distance between the user's upper body weight and the knee rotation axis. The velocity gain **507,** static torque gain **506,** and angle-dependent torque gain **508** are all summed together.

Finally, the Independent Component **509** is added to the three other arms of the controller. While the Independent Component **509** is a feedforward term which is fed through a gain kₜ **510** like the static torque **503** or the Angle Dependant Component **519,** this one does not have an explicit dependency to a gait control parameter like the actuator angle **505** or the Phase detection engine **502,** but is more heuristic in nature and accounts for specific actuator behaviours that are not properly accounted for by the three main branches of the controller structure. Typically, these behaviours are driven from specific user need and are not fundamentally part of physiological gait *per se.* For example, when maintaining the knee in full extension in swing phase is desired, the independent term is used to make the knee resistance increase to a level where the knee will remain in full extension, without any velocity term contribution. Similarly, it is found desirable to increase the actuator resistance prior to foot strike to mitigate any delays observed in ramping up the actuator resistance and user perceived knee buckling at initial loading, which is performed using the independent component **509,** as velocity is also null in this case and the knee is operating in swing phase. Another example where the independent component **509** is used to directly affect the actuator behaviour is in stairs ascent foot placement management. As the knee flexion angle at foot strike is much higher in stairs ascent then in level ground or ramps ambulation, there is a need to stop the swing extension cycle to allow the user to step on the upcoming step, when climbing stairs step-over-step. When the appropriate actuator angle is reached, the high-level stairs ascent management will generate a short increase in actuator resistance to stop the extension motion, allowing the user to step on the flexed knee or kick-it to full extension in the case where transition back to walking is required.

The resulting sum of the four different types of contribution to the controller effort is then fed to the low-pass filter **511** block. The low-pass filter **511** characteristics are dynamically adjusted as a function of the Phase and Subphase information **512** provided by the intent management section of the control system of Figures 4A and 4B, allowing optimal filtering and minimal latency on actuator reaction. For example, swing phase does not show high-bandwidth set-point changes and a uniform behaviour. It is then addressed with a single set of filter parameters. On the other hand, stance phase in most locomotion activities is observed to present high-bandwidth set-point changes due to the user interaction with the environment. Multiple parameter sets are then used in stance phase to ensure obtaining the optimal filter response time for all various situations. One particular low-pass filter embodiment that is found to be particularly suited for the task at hand is the second order Butterworth filter. While not being a limitative embodiment, this filter is known to present optimal response in the pass band with minimum latency, which is of value in the context of a real-time system.

Use of the low-pass filter on the setpoint allows getting rid of the high frequency components and smoothing out the input signal for control purposes. High-frequency components are typically generated by sensor noise, which are propagated and amplified through the different control scheme components. The high-frequency content also includes discontinuities found in the static torque (**503**), Angle Dependant Component (**519**) constitutive terms of the setpoint signal, as well as the independent component (**509**). While specific considerations can be taken to minimize the discontinuous parts generated by the independent contributors to the control signal, some are unavoidable in certain scenarios and use of the low-pass filter allows uniformizing the control signal as a whole, without requiring a heuristic approach. Finally, the Luenberg observer (**504**) also has a contribution to the high-frequency components since it essentially acts as a controller on the predicted and measured actuator positions with a fairly high gain. Attenuation of the aforementioned high-frequency noise and discontinuities removes audible noise generated in the actuator, contributes to attenuating possible chatter in the control loop, and provides a smoother experience to the user. Specific attention is required in compromising between having too small of a bandwidth and the delay of the filter, but the chosen design allows for minimal ringing artifacts in both the pass and stop-band, whilst still allowing for a steep drop-off.

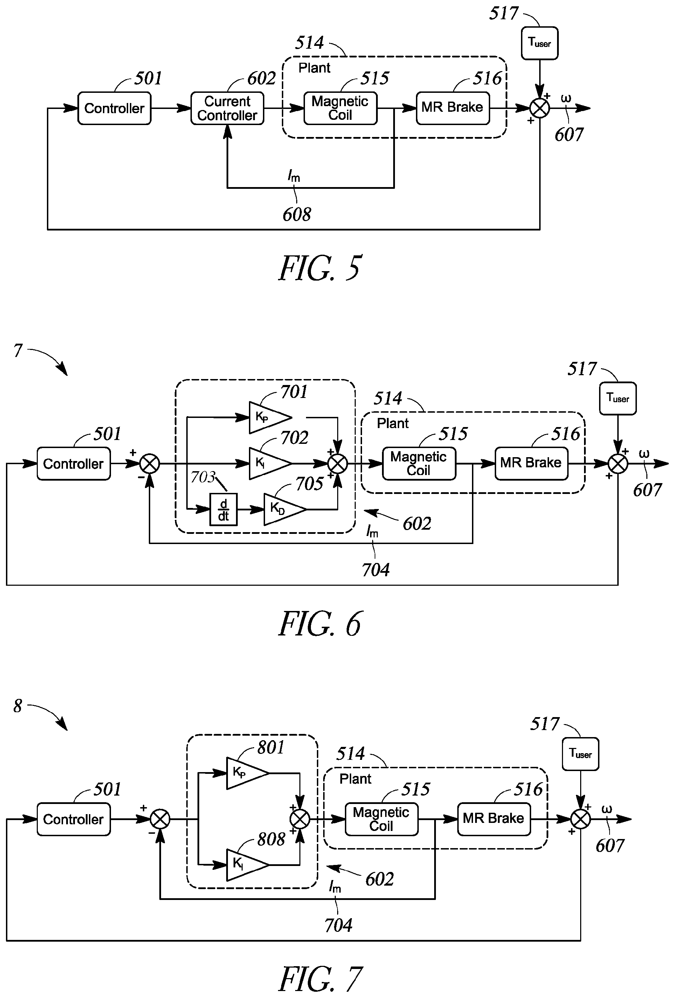

Figure 5 illustrates a simplified representation of the prosthetic knee joint using a MR actuator control system. As mentioned above, as the implementation of the current control loop is independent of the outer control loop, simplifying the more detailed block diagram of Figure 4 improves readability. It is to be noted that the block diagrams of Figure 4 and Figure 5 are functionally equivalent. Again, the plant **514** includes the MR actuator as integrated in the prosthetic knee joint and is modeled as a magnetic coil **515** and a MR brake **516.** Magnetic coil **515** is functionally connected to the current controller **602** through the current set-point signal *Iᵣ* and the measured current signal *Iₘ*. MR actuator combined with the current control system is subject the interactions with the user and the environments, which are modeled through the perturbation torque input **517.** Interactions between the plant **514** under the control of the current controller **602** and external perturbations results in a knee joint velocity **607,** which is feedback to the outer loop controller **501,** responsible for calculating the required changes to the current set-point. In one implementation, the controller **501** can be a velocity controller, such as the one described in Figure 4 above. In other implementations, the controller **501** can be a different type of controller such as, for example, an impedance controller, a position controller, a torque controller or a combination of any of these. Where a controller other than a velocity controller is used, feedback variable **607** may require processing to make it possible to feed it back directly. The present disclosure is associated with the definition and implementation of the current controller **602.**

Figure 6 further details the structure of a current controller **7** for controlling a magnetic coil, such as the one found in a brushless DC motor or solenoid. It is to be noted that the other blocks provided in the block diagram of Figure 6 are equivalent to their counterparts from Figure 5 and will not be further detailed. Further, as described in Figure 5 above, in one implementation the controller **501** can be a velocity controller, such as the one described in Figure 4 above. In other implementations, the controller **501** can be a different type of controller, such as, for example, an impedance controller, a position controller, a torque controller or a combination of any of these. One typical approach to implement closed-loop current control for a magnetic coil is to use a Proportional-Integral-Derivative (PID) controller. While many implementations are known to this particular controller type, Figure 6 illustrates a parallel implementation, where the three terms are implemented independently of each other based on the calculated error between the current set-point issued from the outer loop control scheme and the measured current **704,** which is found from sensing the actual current circulating in the magnetic coil at any given time. The proportional term **701** directly applies a gain K_{P} on the calculated error value, hence amplifying the observed error to drive the control signal harder when the difference between the measured current and the set-point is observed to be large. The integral term **702** applies a gain K_{I} to the integrated error over time. The integral term is similar in nature to the proportional term as they are both applied at the error signal directly, but the integral term is more sensitive to small amplitude error, as it integrates the error over time, growing a small error to bigger one if they are not reducing in time. The derivative term **705** applies a gain K_{D} to the time derivative **703** of the error signal, allowing for a faster reaction to small changes in trend in the error signal. Contributions from all three branches of the Proportional-Integral-Derivative controller **7** are then summed together to form the command signal provided to the magnetic coil drive circuitry, which is not represented in this figure for sake of simplicity. Resulting current circulating in the magnetic coil **515** is measured and provided as feedback *I_{M}* **704.** The feedback value is then subtracted from the current loop set-point value to form the error signal as mentioned above.

Due to the nature of the plant at hand in this type of application and the use of the current as a control variable for controlling the magnetic field present in the MR actuator, use of a derivative term is known not to yield significant benefits and can optionally be removed from the current controller **7** to simplify the implementation. This embodiment of the inner control scheme is generally referred to as a Proportional-Integral controller **8** and its implementation in the current system is presented in Figure 7. The structure is the same as observed for the Proportional-Integral-Derivative controller **7** presented in Figure 6, but this time only two terms are implemented in parallel. The proportional term **801** and its gain K_{P} and the integral term **808** and its gain K_{I}. In one implementation, the controller **501** can be a velocity controller, such as the one described in Figure 4 above. In other implementations, the controller **501** can be a different type of controller, such as, for example, an impedance controller, a position controller, a torque controller, or a combination of any of these.

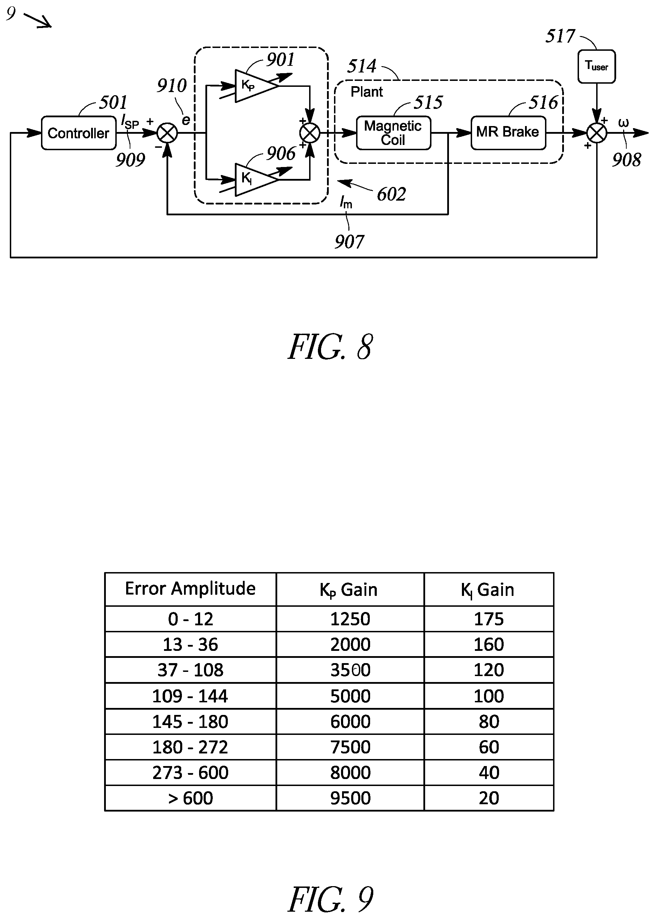

Figure 8 adds to the block diagram of Figure 7 by refining the form in which the proportional and integral gains are implemented in the current controller **9.** The current control loop takes its set-point from the high-level velocity controller **501,** which determines the MR actuator current set-point based on the overall application objective and the knee joint velocity feedback. Current set-point I_{SP} **909** is compared with the measured current Iₘ **907** circulating in the magnetic coil **515** at any given instant to form the error signal e **910.** The error signal is directly amplified by the proportional gain K_{P} **901** in one arm of the control scheme, while the time-integrated value of the error signal is multiplied by the integral gain K_{I} **906.** Contribution of both arms of the control scheme are summed together to form the command signal pushed to the magnetic coil **515** through the coil driver circuitry. In one implementation, the controller **501** can be a velocity controller, such as the one described in Figure 4 above. In other implementations, the controller **501** can be a different type of controller such as, for example, an impedance controller, a position controller, a torque controller, or a combination of any of these.

Unlike the static implementation of the PI controller illustrated in Figure 7 and prior figures, the PI closed-loop current controller embodiment of Figure 8 relies on variable proportional and integral gains, as noted by the arrows crossing the gain blocks **901** and **906.** Determination of the K_{P} and K_{I} gain values is performed in real-time as part of the discrete time implementation of the control scheme. As part of the control loop, the error e **910** is calculated at each cycle. Based on the value of the error at that time, the values of the proportional and integral gains are retrieved from a look-up table similar to the one presented in Figure 9. Once the gains are retrieved, it is possible to compute the control signal as all variables are known.

As mentioned above, appropriate selection of the gains allows minimizing the current rise time and ensures that a MR actuator response is fast as possible, which is known to improve performance and user experience. On the other hand, attention must be directed at not using too high of gains when the error is small, as this will cause current oscillation or overshoot, which will negate the benefits of ensuring a fast rise time. In that context, the K_{P} gains are populated in the look up table such that higher gain values are used when the error is large, forcing a more aggressive system response and increased controller action. When the error reduces, the gains are lowered to prevent excessive controller action, leading to poor tracking performance or instability. On the other hand, the K_{I} gains follow an opposite trend, mainly showing lower values when the error is large and larger values when the error is small. These trends are based on the impact of the respective gains on the system response. Using large K_{I} gain values when the error is larger would lead to reduced performance, as the time integration of the signal would result in a large value, which would then require the error to show the opposite sign for a significant amount of time to balance out its effect. In that context, when the error is small, a larger gain value allows the integrator to cause controller action to minimize the error over a smaller timeframe. The proportional gain K_{P} does not act considering the timeframe, as it is not associated with a time integration process, just a direct correction of the control output. To be optimally effective, applying large corrections when the error is large, and conversely small corrections when the error is small is the logical approach to implement when the whole objective is to minimize the error amplitude at all instants.

Both theoretical and empirical approaches can be used to identify appropriate gains for a PI controller in this type of embodiment, where a gains schedule is established based on the amplitude of the error signal. Apart from the case where a single set of gains is required to meet all performance and convergence criteria for the controller application, use of a discrete gain schedule enables varying the performance targets of the gains for different values of the error amplitude. Gains targeting the optimal response based on the amplitude of the error can be empirically tuned by the submitting the system to step inputs of a size matching the amplitude of the error slot being investigated until the desired response is obtained. Alternatively, theoretical gains calculation methods, like Ziegler-Nichols, can be applied in a general fashion to the system under hand and then scaled across the range of error amplitude.

Similar to the gain schedule, division of the error range into slots must be carefully done, taking into consideration the type of dynamic response desired from the system for each slot, as well as the overall targeted system dynamic response. Use of discrete slots covering a certain range of error values limits the work required to establish a complete schedule, while also presenting a simpler correlation with the end application and its requirements (i.e., which type of dynamic response is desired based on the error amplitude). That being said, it would also be possible to create a continuous gain schedule by interpolating between discrete values previously established. In all cases, any of these strategies can be employed as long as it meets the end application requirements.

The exact number of error amplitude slots is also to be considered in view of the targeted overall system dynamic response and the impact of adding additional slots and/or gains set on the overall control loop performance. As the number of slots increases, gains definition overhead increases, while the benefit on the overall dynamic response is not necessarily increasing. Empirical testing shows that for a fixed overall performance target, improvement in dynamic response is no longer observed once a certain number of slots is reached, no matter how many additional slots are added. In a typical embodiment as the one herein described, eight slots of error amplitude were observed to be the optimal number, as addition of more slots did not yield further performance improvement.

In a similar fashion to the gain schedule where more refined gains are used for smaller error amplitudes, it is possible to divide the error slots such that smaller slots are used when the error is smaller, allowing for a more refined gains schedule to be implemented. While this type of embodiment can yield additional benefits in the form of flexibility, it is not a hard requirement and various other types of error slot patterns can be used without departing from the fundamental concept herein described. It is also possible to establish the error slots pattern using more advanced characterization techniques, to account for the combined operation of the control loop and the plant. For example, it is possible to measure the distribution of the error value while the control system is operating and use the resulting occurrence data to define the number of slots and their boundaries. Error amplitude slots such as the one provided in Figure 9 were obtained using this type of methodology.

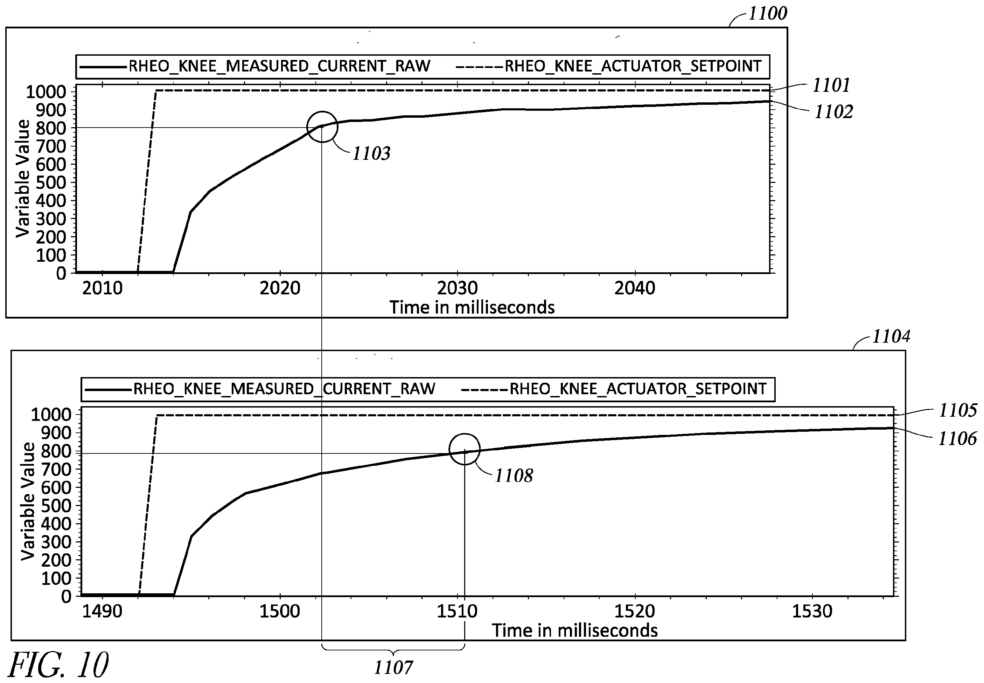

Figure 10 shows graphs illustrating the current response to a step input of a typical MR coil for two different current control approaches, when tested on the same MR actuator and magnetic coil. Top plot **1100** presents the current response **1102** to a controller step input **1101,** where the current response is plotted as current in mA (vertical axis) plotted as a function of time in milliseconds (horizontal axis). At time stamp 2013, the setpoint signal is moved to 1000mA value, creating the step input to the current controller. At time stamp 2014, the current measured in the magnetic coil is observed to start rising under the action of the current controller and the magnetic coil drive circuit. Rapid rise of the current response is observed and presents a mainly linear trend. The consistent trend in the current response in that initial state of the current response is caused by the large current error observed by the controller, causing a large control signal, which in turns causes the magnetic coil drive circuitry to reach saturation, i.e., the magnetic coil drive circuit is putting maximum power through the coil, which causes the current to rise at its maximum rate.

At approximately time stamp 2015, it can be observed that the current rise rate is reduced as the amplitude of the current error is reducing, causing the controller to start using new gains values as per the gain schedule implemented. This trend of reducing current rise rate is continued to be observed as the current error is gradually reducing. Using 80% rise time as performance criteria allows quantifying a rise time characteristic of the performance of the current controller using a gain schedule as per herein described. Data point **1103** shows the 80% rise time point, which is found to happen approximately 9msec after the system was subjected to the step input.

Figure 10 bottom plot **1104** presents the MR coil current response **1106** when a typical current controller not using the gain scheduling scheme herein presented is used and subjected to step input **1105.** Current response **1106** can be observed to be fairly similar to the one observed when using the gain scheduling approach herein described **1102** for the first instant after the step input is created. However, from approximately time stamp 1498, it can be observed that the gain scheduling based current response **1102** starts overtaking the traditional current control approach, as the amplitude of the current is larger, leading to a smaller current error. Again, looking at the 80% current rise as the performance criteria for the current control strategy allows one to measure the current response at point **1108** to be approximately 17 milliseconds ("ms").

Aligning the current response plots for the gain scheduling current control scheme **1100** with the one not using the gain scheduling current control scheme herein disclosed **1104** allows for direct comparison between the performance of the two schemes. Bracket **1107** indicates the time difference required to reach the 80% current rise mark for both methods. As mentioned above, it takes approximately an additional 8 ms while not using the gain scheduling approach to reach 80% of the step input amplitude, which advantageously makes the gain scheduling current scheme to be about 47% faster (8/17) than the more traditional method.

The capacity to increase the current circulating in the magnetic coil of the MR actuator faster directly improves the performance of the lower-limb prosthetic device by allowing it to be more responsive to change of use context, where a sudden demand in braking torque is generated by the high-level controller. One such situation is known to happen when the user is standing at the top of a ramp or staircase and begins to walk downwards. As the user puts his or her prosthetic foot down, the combination of the forward momentum, user weight and increased leverage over the knee prosthesis actuator caused by the downward surface inclination will then create a large demand to brake the knee motion. Sudden increases in knee torque must be achieved through increase in magnetic field, which in turn is based on the current controller's capacity to quickly change the current circulating in the magnetic coil.

In that context, the current control scheme using gain scheduling herein described is observed to be almost 50% faster than other known implementations in achieving 80% of the set-point when the system is submitted to a large change in current set-point.

While certain embodiments have been described, these embodiments have been presented by way of example only and are not intended to limit the scope of the disclosure. Indeed, the novel methods and systems described herein may be embodied in a variety of other forms. Furthermore, various omissions, substitutions and changes in the systems and methods described herein may be made without departing from the disclosure. Accordingly, the scope of the present disclosure is defined only by reference to the appended claims.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable sub-combination. Moreover, although features may be described above as acting in certain combinations, one or more features from a claimed combination can, in some cases, be excised from the combination, and the combination may be claimed as a sub-combination or variation of a sub-combination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present disclosure is not intended to be limited by the specific disclosures of preferred embodiments in this section or elsewhere in this specification, and may be defined by claims as presented in this section or elsewhere in this specification or as presented in the future. The language of the claims is to be interpreted broadly based on the language employed in the claims and not limited to the examples described in the present specification or during the prosecution of the application, which examples are to be construed as non-exclusive.

Of course, the foregoing description is that of certain features, aspects and advantages of the present invention, to which various changes and modifications can be made without departing from scope of the present invention. Moreover, the devices described herein need not feature all of the objects, advantages, features and aspects discussed above. Thus, for example, those of skill in the art will recognize that the invention can be embodied or carried out in a manner that achieves or optimizes one advantage or a group of advantages as taught herein without necessarily achieving other objects or advantages as may be taught or suggested herein. In addition, while a number of variations of the invention have been shown and described in detail, other modifications and methods of use, which are within the scope of this invention, will be readily apparent to those of skill in the art based upon this disclosure. It is contemplated that various combinations or sub-combinations of these specific features and aspects of embodiments may be made and still fall within the scope of the invention. Accordingly, it should be understood that various features and aspects of the disclosed embodiments can be combined with or substituted for one another in order to form varying modes of the discussed devices.

## Claims

1. A prosthetic or orthotic device (1), comprising:
a frame configured to house electronics;
an actuator (101) movably coupled to the frame, the actuator configured to rotate in an anterior-posterior direction about a medial-lateral axis, the actuator comprising a magnetorheological fluid and a coil (205, 302, 515) operable to selectively apply a magnetic field to the magnetorheological fluid to vary its viscosity and thereby vary a torsional resistance of the actuator about the medial-lateral axis; and
circuitry configured to control an amplitude of a current applied to the coil, **characterized by** the circuitry configured to employ a gains schedule to accelerate a change in the current amplitude based on an error amplitude between a current set point and a measured current (518, 704, 907) to reduce a response time for varying the torsional resistance of the actuator.

2. The prosthetic or orthotic device of Claim 1, where the actuator is coupled to a proximal portion of the frame.

3. The prosthetic or orthotic device of any of Claims 1-2, wherein the device is a prosthetic knee.

4. The prosthetic or orthotic device of any of Claims 1-3, wherein the gains schedule includes a plurality of proportional gains and a plurality of integral gains.

5. The prosthetic or orthotic device of Claim 4, wherein the gains schedule includes a plurality of discrete gain values that are decreasing or increasing with a change in the error amplitude.

6. The prosthetic or orthotic device of any of Claims 1-5, wherein the circuitry has a Proportional-Integral controller (8).

7. The prosthetic or orthotic device of Claim 5, wherein the plurality of discrete gain values include a plurality of proportional gains and a plurality of integral gains.

8. The prosthetic or orthotic device of Claim 6, wherein a proportional gain of the Proportional-Integral controller decreases when the error amplitude decreases, and wherein an integral gain of the Proportional-Integral controller increases when the error amplitude decreases.

9. The prosthetic or orthotic device of Claim 1 wherein the gains schedule uses discrete ranges of current error amplitude to determine a value of a proportional gain and a value of an integral gain to use.

10. The prosthetic or orthotic device of Claim 9, wherein the ranges of current error values are uniformly distributed.

11. The prosthetic or orthotic device of Claim 9, wherein the ranges of current error values are reducing when an error size reduces.

12. The prosthetic or orthotic device of Claim 4, wherein the gains schedule is continuous and establishes a direct relationship between the current error amplitude and the proportional and integral gains.

13. The prosthetic or orthotic device of any of Claims 1-12, wherein the circuitry is configured to empirically determine gains in the gains schedule.

14. The prosthetic or orthotic device of any of Claims 1-13, wherein the circuitry is configured to determine gains in the gains schedule using a Ziegler-Nichols calculation.

## Patentansprüche

1. Eine Prothesen- oder Orthesenvorrichtung (1), die Folgendes umfasst:
einen Rahmen, der dazu ausgelegt ist, Elektronik aufzunehmen;
einen Aktor (101), der beweglich mit dem Rahmen gekoppelt ist, wobei der Aktor dazu ausgelegt ist, sich in einer anterior-posterioren Richtung um eine medio-laterale Achse zu drehen, wobei der Aktor eine magnetorheologische Flüssigkeit und eine Spule (205, 302, 515) umfasst, die dazu betreibbar ist, selektiv ein Magnetfeld auf die magnetorheologische Flüssigkeit anzuwenden, um deren Viskosität zu verändern und dadurch einen Torsionswiderstand des Aktors um die medio-laterale Achse zu verändern; und
eine Schaltung, die dazu ausgelegt ist, eine Amplitude eines an die Spule angelegten Stroms zu steuern, **dadurch gekennzeichnet, dass** die Schaltung dazu ausgelegt ist, einen Verstärkungsplan zu verwenden, um eine Änderung der Stromamplitude basierend auf einer Fehleramplitude zwischen einem Stromsollwert und einem gemessenen Strom (518,704,907) zu beschleunigen, um die Reaktionszeit zum Verändern des Torsionswiderstands des Aktors zu verringern.

2. Die Prothesen- oder Orthesenvorrichtung nach Anspruch 1, wobei der Aktor mit einem proximalen Abschnitt des Rahmens gekoppelt ist.

3. Die Prothesen- oder Orthesenvorrichtung nach einem der Ansprüche 1-2, wobei die Vorrichtung ein Prothesenkniegelenk ist.

4. Die Prothesen- oder Orthesenvorrichtung nach einem der Ansprüche 1-3, wobei der Verstärkungsplan eine Mehrzahl von Proportionalverstärkungen und eine Mehrzahl von Integralverstärkungen umfasst.

5. Die Prothesen- oder Orthesenvorrichtung nach einem der Ansprüche 4, wobei der Verstärkungsplan eine Mehrzahl diskreter Verstärkungswerte umfasst, die sich mit einer Änderung der Fehleramplitude verringern oder erhöhen.

6. Die Prothesen- oder Orthesenvorrichtung nach einem der Ansprüche 1-5, wobei die Schaltung einen Proportional-Integral-Regler (8) aufweist.

7. Die Prothesen- oder Orthesenvorrichtung nach Anspruch 5, wobei die Mehrzahl diskreter Verstärkungswerte eine Mehrzahl von Proportionalverstärkungen und eine Mehrzahl von Integralverstärkungen umfasst.

8. Die Prothesen- oder Orthesenvorrichtung nach Anspruch 6, wobei die Proportionalverstärkung des Proportional-Integral-Reglers abnimmt, wenn die Fehleramplitude abnimmt, und wobei die Integralverstärkung des Proportional-Integral-Reglers zunimmt, wenn die Fehleramplitude abnimmt.

9. Die Prothesen- oder Orthesenvorrichtung nach Anspruch 1, wobei der Verstärkungsplan diskrete Bereiche der Stromfehleramplitude verwendet, um einen zu verwendenden Wert einer Proportionalverstärkung und einen Wert einer Integralverstärkung zu bestimmen.

10. Die Prothesen- oder Orthesenvorrichtung nach Anspruch 9, wobei die Bereiche der Stromfehlerwerte gleichmäßig verteilt sind.

11. Die Prothesen- oder Orthesenvorrichtung nach Anspruch 9, wobei die Bereiche der Stromfehlerwerte kleiner werden, wenn sich eine Fehlergröße verringert.

12. Die Prothesen- oder Orthesenvorrichtung nach Anspruch 4, wobei der Verstärkungsplan kontinuierlich ist und eine direkte Beziehung zwischen der Stromfehleramplitude und den Proportional- und Integralverstärkungen herstellt.

13. Die Prothesen- oder Orthesenvorrichtung nach einem der Ansprüche 1-12, wobei die Schaltung dazu ausgelegt ist, Verstärkungen in dem Verstärkungsplan empirisch zu bestimmen.

14. Die Prothesen- oder Orthesenvorrichtung nach einem der Ansprüche 1-13, wobei die Schaltung dazu ausgelegt ist, Verstärkungen in dem Verstärkungsplan unter Verwendung einer Ziegler-Nichols-Berechnung zu bestimmen.

## Revendications

1. Dispositif prothétique ou orthopédique (1), comprenant :
un cadre configuré pour loger des composants électroniques ;
un actionneur (101) couplé de manière mobile au cadre, l'actionneur étant configuré pour tourner dans une direction antérieure-postérieure autour d'un axe médio-latéral, l'actionneur comprenant un fluide magnétorhéologique et une bobine (205, 302, 515)
apte à appliquer sélectivement un champ magnétique au fluide magnétorhéologique afin de faire varier sa viscosité et, de ce fait, de faire varier une résistance en torsion de l'actionneur autour de l'axe médio-latéral ; et
un circuit configuré pour commander une amplitude d'un courant appliqué à la bobine, **caractérisé en ce que** le circuit est configuré pour utiliser une programmation de gains afin d'accélérer une variation de l'amplitude du courant sur la base d'une amplitude d'erreur entre une consigne de courant et un courant mesuré (518, 704, 907), afin de réduire un temps de réponse pour faire varier la résistance en torsion de l'actionneur.

2. Dispositif prothétique ou orthopédique selon la revendication 1, dans lequel l'actionneur est couplé à une partie proximale du cadre.

3. Dispositif prothétique ou orthopédique selon l'une quelconque des revendications 1 à 2, dans lequel le dispositif est une prothèse de genou.

4. Dispositif prothétique ou orthopédique selon l'une quelconque des revendications 1 à 3, dans lequel la programmation de gains comprend une pluralité de gains proportionnels et une pluralité de gains intégraux.

5. Dispositif prothétique ou orthopédique selon l'une quelconque des revendications 4, dans lequel la programmation de gains comprend une pluralité de valeurs de gain discrètes qui diminuent ou augmentent avec une variation de l'amplitude d'erreur.

6. Dispositif prothétique ou orthopédique selon l'une quelconque des revendications 1 à 5, dans lequel le circuit comporte un régulateur proportionnel-intégral (8).

7. Dispositif prothétique ou orthopédique selon la revendication 5, dans lequel la pluralité de valeurs de gain discrètes comprend une pluralité de gains proportionnels et une pluralité de gains intégraux.

8. Dispositif prothétique ou orthopédique selon la revendication 6, dans lequel un gain proportionnel du régulateur proportionnel-intégral
Le contrôleur diminue lorsque l'amplitude d'erreur diminue, et dans lequel un gain intégral du régulateur proportionnel-intégral augmente lorsque l'amplitude d'erreur diminue.

9. Dispositif prothétique ou orthopédique selon la revendication 1, dans lequel la programmation de gains utilise des plages discrètes d'amplitude d'erreur de courant pour déterminer une valeur d'un gain proportionnel et une valeur d'un gain intégral à utiliser.

10. Dispositif prothétique ou orthopédique selon la revendication 9, dans lequel les plages de valeurs d'erreur de courant sont réparties uniformément.

11. Dispositif prothétique ou orthopédique selon la revendication 9, dans lequel les plages de valeurs d'erreur de courant se réduisent lorsqu'une grandeur d'erreur diminue.

12. Dispositif prothétique ou orthopédique selon la revendication 4, dans lequel la programmation de gains est continue et établit une relation directe entre l'amplitude d'erreur de courant et les gains proportionnel et intégral.

13. Dispositif prothétique ou orthopédique selon l'une quelconque des revendications 1 à 12, dans lequel le circuit est configuré pour déterminer empiriquement les gains dans la programmation de gains.

14. Dispositif prothétique ou orthopédique selon l'une quelconque des revendications 1 à 13, dans lequel le circuit est configuré pour déterminer les gains dans la programmation de gains au moyen d'un calcul de Ziegler-Nichols.
